Europäisches Patentamt

**European Patent Office**

Office européen des brevets

(19)

(11) Veröffentlichungsnummer : **0 205 634**
**B1**

(12) **EUROPÄISCHE PATENTSCHRIFT**

(45) Veröffentlichungstag der Patentschrift :
02.03.88

(51) Int. Cl.⁴ : **A 23 L  2/38**

(21) Anmeldenummer : **85107798.2**

(22) Anmeldetag : **24.06.85**

(54) Getränk, enthaltend Fructose, Vitamin C, Chinin und/oder deren Austauschstoffe.

(43) Veröffentlichungstag der Anmeldung :
**30.12.86 Patentblatt 86/52**

(45) Bekanntmachung des Hinweises auf die Patenterteilung : **02.03.88 Patentblatt 88/09**

(84) Benannte Vertragsstaaten :
**AT BE CH DE FR GB IT LI LU NL SE**

(56) Entgegenhaltungen :
**EP-A- 0 000 077**
**DE-A- 3 115 348**
**FR-A- 2 081 361**
**US-A- 3 958 017**

(73) Patentinhaber : **Bannes, Manfred**
**Rebblick 5**
**D-7595 Sasbachwalden (DE)**

(72) Erfinder : **Bannes, Manfred**
**Rebblick 5**
**D-7595 Sasbachwalden (DE)**

(74) Vertreter : **Vogel, Georg**
**Hermann-Essig-Strasse 35**
**D-7141 Schwieberdingen (DE)**

EP 0 205 634 B1

Jouve, 18, rue St-Denis, 75001 Paris, France

**Beschreibung**

Die Erfindung betrifft ein Getränk, das pro Liter etwa 40 gr. bis etwa 400 gr. Fructose, etwa 0,4 gr. bis etwa 4 gr. Vitamin C und etwa 20 mg bis etwa 80 mg Chinin enthält.

Ein Getränk dieser Art ist aus der DE-A 31 15 348 bekannt. Dieses bekannte Getränk wird zur Reduzierung des Blutalkoholgehaltes verwendet, wobei der Zusatz von Chinin den süßen Geschmack des hohen Fructosegehaltes gänzlich aufhebt und dem Getränk einen leicht bitteren, herben Geschmack verleiht.

Es hat sich gezeigt, daß beim Genuß von Alkohol dem Körper viel Wasser mit Mineralien entzogen werden, was das Befinden der Person stark beeinträchtigt. Durch den Abbau des Blutalkoholgehaltes mit dem bekannten Getränk wird aber das durch den Entzug der Mineralien verschlechterte Befinden nicht verbessert.

Es ist Aufgabe der Erfindung, das bekannte Getränk zur Blutalkoholsenkung so zu verbessern, daß auch das mit dem Entzug von Wasser und Mineralien verschlechterte Befinden merklich verbessert wird.

Dies wird nach der Erfindung dadurch erreicht, daß das Getränk zusätzlich verträgliche Minerale mit einer Konzentration von etwa 20 mg bis etwa 80 mg Natriumchlorid, etwa 0,09 mg bis etwa 0,36 mg Trikaliumcitrat, etwa 0,014 mg bis etwa 0,056 mg Magnesiumcarbonat und etwa 0,017 mg bis etwa 0,067 mg Kalziumcarbonat pro Liter enthält.

Die so dosierten Mineralzusätze sind ausreichend, um den Mineralienverlust im Körper auszugleichen und das Befinden der Person zu verbessern. Die Zusätze sind mit diesen Mengen auf die mit dem Ausgangsgetränk erzielte Alkoholgehaltabsenkung abgestimmt, so daß bei größeren Mengen von eingenommenem Getränk nicht zuviele Mineralstoffe aufgenommen werden.

Das Getränk kann durch zusätzliche Anteile von Kaliumchlorid, Natriumcitrat, Magnesiumcitrat, Kaliumcitrat, Kaliumglukonat angereichert werden.

Durch den Zusatz von Lemonextrakt und/oder Zitronenextrakt läßt sich der Geschmack des Getränkes verbessern.

Die Zusätze werden bevorzugt in kohlensäurehaltiges Wasser gegeben.

Anstelle von Chinin können auch Austauschstoffe, wie Naringin oder der Extrakt von Enzianschlempe, verwendet werden.

Die folgenden Beispiele erläutern das erfindungsgemäße Getränk.

## Beispiel 1

Durch Mischen der entsprechenden Substanzen mit Wasser wird ein Getränk hergestellt, das pro Liter enthält :

190 g Fructose, 2,5 g Vitamin C, 4 g Zitronensäure, 0,8 g natürliches Zitronenaroma, 50 mg Natriumchlorid, 0,25 mg Trikaliumcitrat, 0,035 mg Magnesiumcarbonat und 0,042 mg Kalziumcarbonat.

Das Getränk wurde bei 10 Alkoholtrinkern während des Alkoholgenusses auf seine Akzeptanz geprüft. 9 von 10 Personen weigerten sich, aufgrund des widerlich süßen Geschmackes mehr als 100 ml des Getränkes zu sich zu nehmen.

Es wurde nun ein Getränk gemäß der vorliegenden Erfindung hergestellt durch Mischen der angegebenen Substanzen mit Wasser. Das fertige Getränk enthielt pro Liter 190 g Fructose, 2,5 g Vitamin C, 46 mg Chinin und 19 g Lemonenextrakt. Der herbe, bittere Geschmack wurde nun von allen 10 Alkoholtrinkern voll akzeptiert, da der Geschmack den Alkoholgenuß nicht beeinträchtigte. Alle Personen tranken mindestens 500 ml dieses Getränkes.

## Beispiel 2

Es wurde ein Getränk gemäß der vorliegenden Erfindung hergestellt, indem in 500 l kohlensäurehaltiges Wasser unter Rühren 240 kg Fructosesirup 90 %, 3 kg Vitamin C, 20 kg Lemonenextrakt, 50 g Chinin, 50 g Natriumchlorid, 0,25 g Trikaliumcitrat, 0,035 g Magnesiumcarbonat und 0,042 g Kalziumcarbonat gemischt wurden. Dann wurde mit kohlensäurehaltigem Wasser auf 1.000 l aufgefüllt und das Getränk in Flaschen zu je 0,33 l abgefüllt.

Das bitter herb schmeckende Getränk wurde von 20 alkoholtrinkenden Personen während des Alkoholgenusses getrunken (ein bis zwei Flaschen pro Person) und als außerordentlich wohlschmeckend und bekömmlich beurteilt.

## Beispiel 3

Einer Gruppe von 10 Personen wurde nach 3-stündigem Fasten pro Person 300 ml Whisky, verdünnt mit 300 ml Wasser, innerhalb von 30 Minuten zum Trinken gegeben. Innerhalb der nächsten 3 Stunden wurde alle 30 Minuten der Blutalkoholspiegel und dadurch die Abbaurate des Alkohols bestimmt. Die Abbaurate betrug im Mittel 16,5 mg /100 ml/Stunde.

Nach 3 Stunden nahmen die Personen in einem Zeitraum von 15 Minuten 500 ml eines erfindungsgemäßen, kohlensäurehaltigen Getränkes mit folgender Zusammensetzung zu sich :

Fructose 200 g/l
Vitamin C 3 g/l
Chinin 46 mg/l
Lemonenextrakt 22 g/l

Natriumchlorid 50 mg/l
Trikaliumcitrat 0,25 mg/l
Magnesiumcarbonat 0,035 mg/l
Kalziumcarbonat 0,042 mg/l

Sonstige Zucker : Glucose 15 g/l, Saccharose 15 g/l.

Alle Personen beurteilten den mild herben Geschmack des Getränkes als ausgezeichnet. Wieder wurde alle 30 Minuten der Blutalkoholspiegel bestimmt und daraus die Geschwindigkeit des Alkoholabbaues berechnet: Der Alkoholabbau war nun im Mittel beträchtlich gesteigert auf 21,1 mg/100 ml/Stunde.

## Patentansprüche

1. Getränk mit etwa 40 gr. bis etwa 400 gr. Fructose, etwa 0 4 gr. bis etwa 4 gr. Vitamin C und etwa 20 mg bis etwa 80 mg Chinin pro Liter, dadurch gekennzeichnet, daß es zusätzlich verträgliche Minerale mit einer Konzentration von etwa 20 mg bis etwa 80 mg Natriumchlorid, etwa 0,09 mg bis etwa 0,36 mg Trikaliumcitrat, etwa 0,014 mg bis etwa 0,056 mg Magnesiumcarbonat und etwa 0,017 mg bis etwa 0,067 mg Kalziumcitrat pro Liter enthält.

2. Getränk nach Anspruch 1, dadurch gekennzeichnet, daß es zusätzlich Anteile von Kaliumchlorid, Natriumcitrat, Magnesiumcitrat, Kalziumcitrat, Kaliumglukonat enthält.

3. Getränk nach Anspruch 1 oder 2, dadurch gekennzeichnet, daß es zusätzlich Lemonextrakt enthält.

4. Getränk nach einem der Ansprüche 1 bis 3, dadurch gekennzeichnet, daß es zusätzlich Zitronenextrakt enthält.

5. Getränk nach einem der Ansprüche 1 bis 4, dadurch gekennzeichnet, daß es zusätzlich Kohlensäure enthält.

6. Getränk nach einem der Ansprüche 1 bis 5, dadurch gekennzeichnet, daß anstelle Chinin Austauschstoffe wie Naringin oder der Extrakt von Enzianschlempe, verwendet sind.

## Claims

1. A drink having about 40g to about 400g fructose, about 0,4 g to about 4 g vitamin C and about 20 mg to about 80 mg quinine per litre, charaterised in that it additionally contains compatible minerals with a concentration of about 20 mg to about 80 mg sodium chloride, about 0,09 mg to about 0,36 mg tripotassium citrate, about 0,014 mg to about 0,056 mg magnesium carbonate and about 0,017 mg to about 0,067 mg calcium carbonate per litre.

2. A drink according to claim 1, characterised in that it additionally contains amounts of potassium chloride, sodium citrate, magnesium citrate, calcium citrate and potassium gluconate.

3. A drink according to claim 1 or claim 2, characterised in that it additionally contains lemon extract.

4. A drink according to one of claims 1 to 3, characterised in that it additionally contains citrus fruit extract.

5. A drink according to one of claims 1 to 4, characterised in that in additionally contains carbonate dioxide.

6. A drink according to one of claims 1 to 5, characterised in that instead of quinine substitute substances are used such as naringin or the extract of gentian residual liquid.

## Revendications

1. Boisson renfermant environ 40 g à environ 400 g de fructose, environ 0,4 g à 4 g de vitamine C et environ 20 mg à environ 80 mg de quinine par litre, caractérisée par le fait qu'elle renferme en outre des produits minéraux acceptables, présents en une concentration d'environ 20 mg à environ 80 mg de chlorure de sodium, environ 0,09 mg à environ 0,36 mg de citrate tripotassique, environ 0,014 mg à environ 0,056 mg de carbonate de magnésium et environ 0,017 mg à environ 0,067 mg de carbonate de calcium par litre.

2. Boisson selon la revendication 1, caractérisée par le fait qu'elle renferme en outre des fractions constituées par du chlorure de potassium, du citrate de sodium, du citrate de magnésium, du citrate de calcium, du gluconate de potassium.

3. Boisson selon l'une des revendications 1 ou 2, caractérisée par le fait qu'elle renferme en outre de l'extrait de limon.

4. Boisson selon l'une quelconque des revendications 1 à 3, caractérisée par le fait, qu'elle renferme en outre de l'extrait de citron.

5. Boisson selon l'une quelconque des revendications 1 à 4, caractérisée par le fait qu'elle renferme en outre de l'acide carbonique.

6. Boisson selon l'une quelconque des revendications 1 à 5, caractérisée par le fait qu'elle renferme au lieu de quinine des produits de remplacement, tels que la naringine ou de l'extrait de gentiane.